(19) Europäisches Patentamt
European Patent Office
Office européen des brevets

(11) Numéro de publication : **0 544 740 B1**

(12) **FASCICULE DE BREVET EUROPEEN**

(45) Date de publication du fascicule du brevet :
06.07.94 Bulletin 94/27

(51) Int. Cl.⁵ : **C07C 59/84,** C07C 51/487, C07B 57/00

(21) Numéro de dépôt : **91914913.8**

(22) Date de dépôt : **19.08.91**

(86) Numéro de dépôt international :
**PCT/FR91/00670**

(87) Numéro de publication internationale :
**WO 92/03404 05.03.92 Gazette 92/06**

(54) **PROCEDE DE TRANSFORMATION DE L'ACIDE (BENZOYL-3 PHENYL)-2 PROPIONIQUE-R(-) EN ISOMERE S(+).**

(30) Priorité : **20.08.90 FR 9010460**

(43) Date de publication de la demande :
**09.06.93 Bulletin 93/23**

(45) Mention de la délivrance du brevet :
**06.07.94 Bulletin 94/27**

(84) Etats contractants désignés :
**AT BE CH DE DK ES FR GB GR IT LI LU NL SE**

(56) Documents cités :
**EP-A- 0 362 476**
**FR-M- 296**
**GB-A- 2 154 233**
**US-A- 4 831 147**

(72) Inventeur : **BERTRAND, Claude**
**28, avenue Jean-Giono**
**F-91250 Saint-Germain-les-Corbeil (FR)**
Inventeur : **FOUQUE, Elie**
**90, avenue de Bonneuil**
**F-94100 Saint-Maur-des-Fossés (FR)**
Inventeur : **LE FUR, Isidore**
**7, rue Jeanne-d'Arc**
**F-94320 Thiais (FR)**
Inventeur : **RICHARD, Jean-Paul**
**307 Arenimink Drive**
**Royersford, PA 19468-1151 (US)**

(74) Mandataire : **Pilard, Jacques**
**RHONE-POULENC RORER S.A.**
**Direction Brevets (t144)**
**20 avenue Raymond Aron**
**F-92165 Antony Cédex (FR)**

(73) Titulaire : **RHONE-POULENC RORER S.A.**
**20, avenue Raymond Aron**
**F-92160 Antony (FR)**

EP 0 544 740 B1

## Description

La présente invention concerne un procédé de transformation de l'acide (benzoyl-3 phényl)-2 propionique-R(-) en isomère S(+).

L'acide (benzoyl-3 phényl)-2 propionique (ou kétoprofène) présente des propriétés antiinflammatoires, analgésiques et/ou antipyrétiques particulièrement intéressantes.

Le kétoprofène, sous forme racémique, est constitué d'un mélange équimoléculaire des énantiomères S(+) et R(-).

Alors que chez l'animal, il n'existe pas de différences significatives entre le kétoprofène racémique et l'isomère S(+) [kétoprofène-S(+)], il a été montré que, chez l'homme, le kétoprofène-S(+) constitue la forme active du kétoprofène et que l'isomère R(-) n'est pas converti en isomère S(+).

Il est particulièrement avantageux de pouvoir disposer de kétoprofène-S(+) pratiquement exempt de l'isomère R(-).

Le kétoprofène-S(+) peut être obtenu par dédoublement du kétoprofène racémique soit par des méthodes physico-chimiques (chromatographie liquide à haute performance avec une phase chirale) soit par formation d'un sel avec une base optiquement active. Ainsi le brevet français FR 296M (qui est en fait un certificat d'addition au brevet français FR 6444M) concerne un procédé chimique complexe de préparation du kétoprofène-(S).

Le brevet européen EP 362 476 enseigne le dédoublement d'acides aryl-2 propioniques (ibuprofène, kétoprofène et fluorbiprofen) à partir de sels avec des bases optiquement actives choisies parmi la phényléthylamine, la quinine et la cinchonidine. Dans le cas du kétoprofène, le dédoublement est décrit à partir du sel avec la phényléthylamine.

Ces procédés conduisent à l'obtention de l'isomère S(+) et de l'isomère R(-), ce dernier n'ayant pas d'utilité propre. Il est donc particulièrement important de pouvoir disposer d'un procédé qui permette de transformer le kétoprofène-R(-) en kétoprofène S(+).

Il a maintenant été trouvé, et c'est ce qui fait l'objet de la présente invention, que le kétoprofène-R(-) peut être transformé soit in situ pendant le dédoublement du kétoprofène racémique en utilisant notamment l'agent de dédoublement comme agent de transformation soit à partir des filtrats de dédoublement, après avoir éventuellement éliminé la base chirale, par traitement avec une base forte. La soude peut être utilisée comme base forte.

Selon l'invention, le procédé est mis en oeuvre in situ en utilisant comme agent de dédoublement une base chirale comme la cinchonidine et comme solvant une cétone, telle que la méthylisobutylcétone ou un alcool tel que l'éthanol. Il est avantageux d'utiliser la méthylisobutylcétone qui permet d'opérer à des températures élevées. Pour obtenir un rendement amélioré, il est particulièrement avantageux de provoquer la cristallisation du sel désiré majoritaire (sel de cinchonidine avec le kétoprofène-S(+)) par concentration de la solution sous pression réduite.

Généralement, on utilise une mole de cinchonidine par mole de kétoprofène.

Généralement, l'opération de concentration sous pression réduite est réglée de telle manière que la température d'ébullition soit constante et voisine de 100°C pendant toute la durée de la concentration. Les cristaux du sel de kétoprofène-S(+)qui se forment sont séparés par filtration à chaud.

La transformation peut aussi être réalisée indépendamment de la cristallisation à partir des eaux-mères de cristallisation après avoir éliminé la base chirale utilisée comme agent de dédoublement en utilisant une base achirale telle que la soude.

Pour la mise en oeuvre du procédé sur les eaux-mères de cristallisation, on effectue d'abord le dédoublement du kétoprofène racémique en utilisant de 0,5 à 1 mole de cinchonidine par mole de kétoprofène. Après séparation par filtration du sel de cinchonidine avec le kétoprofène (majoritairement le kétoprofène-S(+)), les eaux-mères sont traitées par une solution aqueuse d'une base minérale forte telle que la soude après libération et séparation de la cinchonidine soit sous forme de base soit sous forme de sel. Le kétoprofène (majoritairement le kétoprofène-R(-)), sous forme de sel de sodium, reste en solution aqueuse basique qui est chauffée à une température généralement supérieure à 100°C, de préférence voisine de 110°C en suivant l'évolution du titre optique en fonction du temps, le titre optique étant exprimé par le rapport 100 x R/(R+S). La transformation se caractérise par une diminution progressive du titre optique.

Les exemples suivants montrent comment l'invention peut être mise en pratique.

Exemple 1 - Dédoublement - Racémisation

Dans un réacteur de 250 cm3, on introduit 26,5 g de kétoprofène racémique (O,104 mole), 31 g de cinchonidine (0,105 mole) et 120,6 g (ou 151 cm3) de méthylisobutylcétone (1,204 mole). Le mélange est chauffé, sous agitation, à 100°C puis le mélange est concentré sous pression réduite en réglant la pression de telle manière que la température d'ébullition reste constante et égale à 100°C. On distille 97,2 g de méthylisobutylcétone en 8 heures. On agite pendant une nuit à 100°C 45 minutes à 45°C puis verse le mélange réactionnel sur un filtre thermostaté à 100°C. Le gâteau de filtration est lavé par 70 cm3 de la méthylisobutylcétone à 20°C puis séché. On obtient ainsi 19 g de sel de kétoprofène dont le titre optique (S/R+S) est de 88,3 %.

Le rendement est de 58,6 %.

Le bilan sur le kétoprofène conduit aux résultats suivants :

|  | Kétoprofène (g) | Titre optique (R/S) | Kétoprofène | |
|---|---|---|---|---|
|  |  |  | S(+) (g) | R(-) (g) |
| Produit isolé | 8,80 | 11,7/88,3 | 7,77 | 1,03 |
| Eaux-mères | 8,40 | 54,7/45,3 | 3,8 | 4,6 |
| Lavages | 9,30 | 50,2/49,8 | 4,6 | 4,7 |

Exemple 2 - Racémisation par la soude

Dans un réacteur, on introduit 10,4 g de kétoprofène racémique (0,041 mole), 6 g de cinchonidine (0,020 mole) et 28,9 g (ou 36 cm3) de méthylisobutylcétone (0,288 mole). Le mélange est chauffé à 75°C puis est maintenu à cette température jusqu'à dissolution totale puis est refroidi rapidement à 70°C. On amorce la cristallisation avec quelques cristaux de sel de cinchonidine du kétoprofène-S(+), La bouillie obtenue est refroidie en 6 heures de 70°C à 10°C, à raison de -10°C par heure environ. La bouillie est filtrée à une température voisine de 20°C. Le gâteau de filtration est lavé par 15 g de méthylisobutylcétone. On obtient ainsi 7,3 g d'un sel dont la composition est la suivante :
- sel de cinchonidine du kétoprofène-S(+) : 5,9 g (O,O11 mole)
- sel de cinchonidine du kétoprofène-R(-) : 1,4 g (0,002 mole).

Le rendement est de 53,6 %.

Les filtrats ont la composition suivante :
- kétoprofène-S(+) : 2,5 g (0,010 mole)
- kétoprofène-R(-) : 4,5 g (0,018 mole)
- cinchonidine : 2,1 g (0,007 mole).

Au mélange réactionnel, dont la masse est de 53 g, on ajoute, à température ambiante, 22,1 g de soude aqueuse à 10 % (p/p), soit 0,055 mole.

La cinchonidine reste dans la phase organique tandis que le kétoprofène, sous forme de sel de sodium, reste dans la phase aqueuse. La phase aqueuse est extraite par 32 g (ou 40 cm3) de méthylisobutylcétone puis est chauffée au reflux pendant 24 heures à 110-115°C. On détermine l'évolution du titre optique en fonction du temps. Les résultats sont rassemblés dans le tableau suivant :

| Temps (heures) | Titre optique exprimé en kétoprofène 100 x R/(R+S) |
|---|---|
| 0,0 | 64,8 |
| 2,3 | 59,6 |
| 4,6 | 56,8 |
| 21,5 | 50,9 |

Exemple 3 - Racémisation par la soude

Dans un réacteur émaillé de 300 litres, on introduit 175 litres (145,3 kg) d'un filtrat de dédoublement du kétoprofène racémique par la cinchonidine dans l'éthanol dont la composition est la suivante :
- sel du kétoprofène avec la cinchonidine : 23,07 kg (42,04 moles) [pureté optique du kétoprofène (S/S+R) voisine de 30 %]
- éthanol : 155 litres (122,23 kg)

La solution éthanolique est concentrée sous pression réduite (80 mm de mercure ; 10,7 kPa) à 30°C jusqu'à l'obtention d'une masse épaisse à laquelle on ajoute 57,7 litres (50 kg) de toluène puis on distille encore 20 litres de solvant.

A la solution toluénique chauffée à 50°C par un mélange eau-vapeur en double enveloppe, on ajoute 42 litres d'eau distillée et 18,5 litres d'acide chlorhydrique 9,8N. On agite énergiquement jusqu'à ce que la température du mélange réactionnel atteigne à nouveau 50°C. Après arrêt de l'agitation et décantation, la phase aqueuse inférieure est séparée et la phase organique est lavée par de l'acide chlorhydrique 1N.

A la phase organique maintenue à 50°C, on ajoute 35 litres d'eau distillée et 3,1 litres d'acide chlorhydrique 9,8N. On agite énergiquement pendant 10 minutes. Après décantation la phase aqueuse est réunie aux phases aqueuses précédemment séparées.

La phase organique est lavée par 35 litres d'eau distillée contenant 1,5 litre d'acide chlorhydrique 9,8N puis par 30 litres d'eau distillée. Les lavages sont écartés.

A une solution de 16,5 litres de soude 10N (21,9 kg ; 165 moles) dans 16 litres d'eau distillée, on ajoute la solution toluénique précédente. On agite vigoureusement puis on chauffe à 100°C (vapeur vive dans la double enveloppe). On maintient le mélange réactionnel au reflux pendant 12 heures.

Après refroidissement et après décantation, le mélange réactionnel est constitué de 3 phases :
1) une phase inférieure (11,9 kg) laiteuse qui est séparée et écartée,
2) une phase médiane, légèrement jaune (39 kg) qui contient le sel de sodium du kétoprofène,
3) une phase supérieure toluénique incolore.

L'analyse de la phase médiane par CLHP chirale montre que le titre optique (S/S+R) est de 50 % : la racémisation est totale.

A la phase médiane, chauffée à 50°C, on ajoute 20 litres de toluène et 13 litres d'acide chlorhydrique 9,8N. On agite énergiquement. Après décantation, la phase aqueuse est épuisée par 20 litres de toluène.

Les phases toluéniques sont réunies puis concentrées par distillation de 29 litres de toluène sous pression réduite (80 mm de mercure ; 10,7 kPa) à 42°C.

A la solution toluénique concentrée, chauffée à 70°C, on ajoute du cyclohexane de telle manière que le rapport cyclohexanetoluène soit 6-4 (p/p) soit 23 litres de cyclohexane.

On refroidit à 60°C puis on amorce la cristallisation par addition de 50 g de kétoprofène racémique.

Après refroidissement à une température voisine de 15°C, la bouillie est filtrée puis lavée 2 fois par un mélange de 3,5 litres de toluène et 6 litres de cyclohexane.

On obtient ainsi un gâteau de filtration qui représente 9,76 kg en kétoprofène sec et qui est utilisé tel quel dans une nouvelle opération de dédoublement.

Le titre du kétoprofène, déterminé par CLHP non chirale, est de 100 %.

**Revendications**

1. Procédé de transformation de l'acide (benzoyl-3 phényl)-2 propionique-R(-) en isomère S(+) caractérisé en ce que l'on traite le kétoprofène-R(-) par une base.

2. Procédé selon la revendication 1 caractérisé en ce que la transformation du kétoprofène-R(-) en kétoprofène-S(+) est effectuée in situ pendant le dédoublement du kétoprofène racémique.

3. Procédé selon la revendication 1 caractérisé en ce que la transformation du kétoprofène-R(-) en kétoprofène-S(+) est effectuée sur les eaux-mères de cristallisation d'un sel de kétoprofène-S(+).

4. Procédé selon la revendication 2 caractérisé en ce que l'on utilise une base chirale comme agent de transformation et comme agent de dédoublement.

5. Procédé selon la revendication 4 caractérisé en ce que la base chirale est la cinchonidine.

6. Procédé selon la revendication 2 caractérisé en ce que l'on utilise une mole d'agent de transformation par mole de kétoprofène racémique.

7. Procédé selon la revendicatiion 2 caractérisé en ce que l'on utilise comme solvant une cétone telle que la méthylisobutylcétone ou un alcool tel que l'éthanol.

8. Procédé selon la revendication 2 caractérisé en ce que l'on opère à une température voisine de 100°C.

9. Procédé selon la revendication 3 caractérisé en ce que l'on utilise une base forte après élimination éventuelle de l'agent de dédoublement.

10. Procédé selon la revendication 3 caractérisé en ce que la base forte est la soude.

11. Procédé selon la revendication 3 caractérisé en ce que les eaux-mères proviennent du dédoublement du kétoprofène racémique au moyen d'une base chirale en opérant dans un solvant organique.

12. Procédé selon la revendication 11 caractérisé en ce que la base chirale est la cinchonidine.

13. Procédé selon la revendication 11 caractérisé en ce que l'on utilise 0,5 mole de base chirale par mole de kétoprofène.

14. Procédé selon la revendication 11 caractérisé en ce que l'on utilise comme solvant une cétone telle que la méthylisobutylcétone ou un alcool tel que l'éthanol.

15. Procédé selon la revendication 3 caractérisé en ce que l'on utilise un excès molaire de base par rapport au kétoprofène présent dans les eaux-mères.

16. Procédé selon la revendication 3 caractérisé en ce que l'on opère à une température supérieure à 100°C, de préférence comprise entre 110 et 115°C.


## Patentansprüche

1. Verfahren zur Umwandlung der R-(-)-2-(3-Benzoyl-phenyl)-propionsäure in das S-(+)-Isomere, dadurch gekennzeichnet, daß man R-(-)-Ketoprofen mit einer Base behandelt.

2. Verfahren gemäß Anspruch 1, dadurch gekennzeichnet, daß die Umwandlung von R-(-)-Ketoprofen in S-(+)-Ketoprofen in situ während der Racematspaltung des Ketoprofens erfolgt.

3. Verfahren gemäß Anspruch 1, dadurch gekennzeichnet, daß die Umwandlung von R-(-)-Ketoprofen in S-(+)-Ketoprofen an den Kristallisationsmutterlaugen eines S-(+)-Ketoprofensalzes vorgenommen wird.

4. Verfahren gemäß Anspruch 2, dadurch gekennzeichnet, daß man eine chirale Base als Mittel für die Umwandlung und als Mittel für die Spaltung verwendet.

5. Verfahren gemäß Anspruch 4, dadurch gekennzeichnet, daß die chirale Base Cinchonidin ist.

6. Verfahren gemäß Anspruch 2, dadurch gekennzeichnet, daß man ein Mol des Mittels für die Umwandlung je Mol racemisches Ketoprofen verwendet.

7. Verfahren gemäß Anspruch 2, dadurch gekennzeichnet, daß man als Lösungsmittel ein Keton wie Methylisobutylketon oder einen Alkohol wie Ethanol verwendet.

8. Verfahren gemäß Anspruch 2, dadurch gekennzeichnet, daß man bei einer Temperatur um 100°C arbeitet.

9. Verfahren gemäß Anspruch 3, dadurch gekennzeichnet, daß man nach etwaiger Eliminierung des Mittels für die Spaltung eine starke Base verwendet.

10. Verfahren gemäß Anspruch 3, dadurch gekennzeichnet, daß die starke Base Natronlauge ist.

11. Verfahren gemäß Anspruch 3, dadurch gekennzeichnet, daß die Mutterlaugen der Spaltung des racemischen Ketoprofens mit Hilfe einer chiralen Base bei einer Arbeitsweise in einem organischen Lösungsmittel entstammen.

12. Verfahren gemäß Anspruch 11, dadurch gekennzeichnet, daß die chirale Base das Cinchonidin ist.

13. Verfahren gemäß Anspruch 11, dadurch gekennzeichnet, daß man 0,5 Mol chirale Base je Mol Ketoprofen verwendet.

14. Verfahren gemäß Anspruch 11, dadurch gekennzeichnet, daß man als Lösungsmittel ein Keton wie Methylisobutylketon oder einen Alkohol wie Ethanol verwendet.

15. Verfahren gemäß Anspruch 3, dadurch gekennzeichnet, daß man einen molaren Überschuß der Base in Bezug auf das in den Mutterlaugen vorhandene Ketoprofen verwendet.

16. Verfahren gemäß Anspruch 3, dadurch gekennzeichnet, daß man bei einer Temperatur oberhalb von 100°C, vorzugsweise zwischen 110 und 115°C, arbeitet.

## Claims

1. Process for converting [R(-)]-2-(3-benzoylphenyl)propionic acid to the S(+) isomer, characterized in that [R(-)]-ketoprofen is treated with a base.

2. Process according to Claim 1, characterized in that the conversion of [R(-)]-ketoprofen to [S(+)]-ketoprofen is carried out in situ during the resolution of racemic ketoprofen.

3. Process according to Claim 1, characterized in that the conversion of [R(-)]-ketoprofen to [S(+)]-ketoprofen is carried out using the crystallization mother liquors of an [S(+)]-ketoprofen salt.

4. Process according to Claim 2, characterized in that a chiral base is used as converting agent and as resolving agent.

5. Process according to Claim 4, characterized in that the chiral base is cinchonidine.

6. Process according to Claim 2, characterized in that one mole of converting agent is used per mole of racemic ketoprofen.

7. Process according to Claim 2, characterized in that a ketone such as methyl isobutyl ketone or an alcohol such as ethanol is used as solvent.

8. Process according to Claim 2, characterized in that it is carried out at a temperature close to 100°C.

9. Process according to Claim 3, characterized in that a strong base is used after the optional elimination of the resolving agent.

10. Process according to Claim 3, characterized in that the strong base is sodium hydroxide.

11. Process according to Claim 3, characterized in that the mother liquors are obtained from resolution of the racemic ketoprofen by means of a chiral base by working in an organic solvent.

12. Process according to Claim 11, characterized in that the chiral base is cinchonidine.

13. Process according to Claim 11, characterized in that 0.5 mole of chiral base is used per mole of ketoprofen.

14. Process according to Claim 11, characterized in that a ketone such as methyl isobutyl ketone or an alcohol such as ethanol is used as solvent.

15. Process according to Claim 3, characterized in that a molar excess of base is used relative to the ketoprofen present in the mother liquors.

16. Process according to Claim 3, characterized in that it is carried out at a temperature above 100°C, preferably between 110 and 115°C.